# EUROPÄISCHE PATENTSCHRIFT

(11) **EP 1 263 320 B1**
(45) Veröffentlichungstag und Bekanntmachung des Hinweises auf die Patenterteilung: **14.11.2007**
(21) Anmeldenummer: 01909796.3
(22) Anmeldetag: 28.02.2001
(51) Int. Cl.: A61B 5/15

(54) **BLUTLANZETTE MIT HYGIENISCHEM SPITZENSCHUTZ**
BLOOD LANCET WITH HYGIENIC TIP PROTECTION
LANCETTE A PROTECTION DE POINTE HYGIENIQUE

(30) Priorität: 04.03.2000 DE 10010694
(43) Veröffentlichungstag der Anmeldung: 11.12.2002
(60) Teilanmeldung: 04026936.7
(73) Patentinhaber: F.HOFFMANN-LA ROCHE AG, 4070 Basel (CH); Roche Diagnostics GmbH, 68305 Mannheim (DE)
(72) Erfinder: FRITZ, Michael, 68647 Biblis (DE); ARGAUER, Herbert, 92712 Pirk (DE); LIST, Hans, 64754 Hesseneck-Kailbach (DE); WEISS, Thomas, 68307 Mannheim (DE); DECK, Frank, 67150 Niederkirchen (DE); IMMEKUS, Claudio, 79312 Emmendingen (DE)
(74) Vertreter: Jung, Michael
(86) Internationale Anmeldenummer: PCT/EP2001/002198
(87) Internationale Veröffentlichungsnummer: WO 2001/066010

(56) Entgegenhaltungen:
- EP-A1- 0 931 507
- DE-A1- 3 842 317
- DE-B1- 2 803 345
- US-A- 4 635 633
- US-A- 4 794 926
- US-A- 4 889 117
- US-A- 5 636 640

## Beschreibung

Die Erfindung betrifft eine Lanzette enthaltend eine Lanzettennadel mit einer Spitze und einen Lanzettenkörper, der die Lanzettennadel zumindest im Bereich der Spitze vollständig umgibt Zudem betrifft die Erfindung ein Lanzettenmagazin enthaltend mindestens zwei Lanzetten, die jeweils eine Lanzettennadel mit einer Spitze enthalten und die jeweils in einzelnen, voneinander unabhängigen Kammern des Lanzettenmagazins untergebracht sind, wobei jede Kammer zumindest eine Öffnung für den Austritt der Spitze der Lanzettennadel aufweist. Schließlich betrifft die Erfindung die Verwendung eines elastischen Materials als Bestandteil einer Lanzette oder eines Lanzettenmagazins.

Die Untersuchung von Blutproben ermöglicht in der klinischen Diagnostik das frühzeitige und zuverlässige Erkennen von pathologischen Zuständen sowie die gezielte und fundierte Kontrolle von Körperzuständen. Die medizinische Blutdiagnostik setzt stets die Gewinnung einer Blutprobe des zu untersuchenden Individuums voraus. Während in Kliniken und bei niedergelassenen Ärzten oftmals durch eine Venenpunktion mehrere Milliliter Blut einer zu untersuchenden Person für die Analyse gewonnen werden, um damit eine Vielzahl von Labortests durchführen zu lassen, reichen für einzelne Analysen, die gezielt auf einen Parameter gerichtet sind, heutzutage oftmals wenige Mikroliter Blut aus. Solch geringe Blutmengen erfordern keine Venenpunktion. Vielmehr genügt es hier, zur Blutgewinnung durch die Haut z. B. in die Fingerbeere oder das Ohrläppchen der zu untersuchenden Person mit Hilfe einer sterilen, scharfen Lanzette zu stoßen, um so einige wenige Mikroliter Blut für die Analyse zu gewinnen. Insbesondere eignet sich diese Methode, wenn die Analyse der Blutprobe unmittelbar nach der Blutgewinnung durchgeführt werden kann.

Vor allem im Bereich des sogenannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglucosekonzentration, werden Lanzetten und dazu passende Geräte (sogenannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen. Zudem soll die Verwendung von Lanzetten mit Stechhilfen die psychologische Schwelle beim Stechen des eigenen Körpers senken, was vor allem für Kinder, die an Diabetes erkrankt sind und auf regelmäßige Blutglucosetests angewiesen sind, von besonderer Bedeutung ist. Als Beispiele für Lanzetten und Stechhilfen seien die kommerziell erhältlichen Geräte (Stechhilfen) und Lanzetten Glucolet^{®} der Bayer AG und Softclix^{®} der Roche Diagnostics GmbH genannt. Solche Lanzetten und Geräte (Stechhilfen) sind z. B. Gegenstand von WO-A 98/48695, EP-A 0 565 970, US 4,442,836 oder US 5,554,166.

Die Lanzetten des Standes der Technik weisen meist eine metallene Lanzettennadel mit einer Spitze auf, die gegebenenfalls angeschliffen sein kann. Zur leichteren Handhabung der Lanzette und gegebenenfalls zu ihrer Befestigung in einer Stechhilfe ist bei vielen Ausführungsformen meist an die Lanzettennadel ein Kunststofflanzettenkörper aus einem starren, spritzgußfähigen Material angespritzt. Die Spitze der Lanzettennadel ist - im unbenutzten Zustand - zur Sicherstellung ihrer Sterilität von einer Schutzhülle umgeben. Diese besteht in der Regel aus dem selben starren Material wie der eigentliche Lanzettenkörper und bildet meist mit diesem eine Einheit. Die Schutzhülle kann vor der Benutzung der Lanzette vom Lanzettenkörper getrennt und von der Spitze der Lanzettennadel abgenommen werden. Zwischen Lanzettenkörper und Schutzhülle befindet sich zu diesem Zweck meist eine Sollbruchstelle. Nach der Benutzung der Lanzette liegt die Spitze der Lanzettennadel ungeschützt vor und stellt somit eine potentielle Verletzungsquelle für den Benutzer und eventuell andere Personen dar.

Zur Vermeidung einer unbeabsichtigten Verletzung an einer benutzten Lanzettennadel wird den Benutzern meist empfohlen, die Spitze der Lanzettennadel nach Gebrauch in die zuvor abgenommen Schutzhülle zu stecken. Allerdings lehrt die Erfahrung, daß bei weitem nicht alle Benutzer dieser Empfehlung folgen und somit ein großer Anteil benutzter Lanzetten mit ungeschützten Spitzen weggeworfen werden. US 5,304,192 und WO-A 96/02189 schlagen als Lösung für dieses Problem Lanzetten vor, bei denen die Spitze der Lanzettennadel nach Gebrauch in den Lanzettenkörper geschoben oder gezogen werden kann. Da der Lanzettenkörper in diesen Fällen aus einem nicht-elastischen, weitgehend starren oder steifen Material gefertigt ist wird die Spitze der Lanzettennadel zwar im Lanzettenkörper verborgen, nicht jedoch vollkommen hygienisch abgeschirmt, da im Material des Lanzettenkörpers ein Kanal verbleibt, über den die Lanzettenspitze mit der Umgebung in Kontakt steht.

Bei den derzeit kommerziell verfügbaren Systemen erfolgt die Bereitstellung der Lanzetten für die Verwendung in Stechhilfen meist in loser Form. Der Benutzer entnimmt manuell vor jedem Stechvorgang eine Lanzette aus einer Verpackung, beispielsweise einer Pappschachtel oder einer Röhre, in der eine Vielzahl von Lanzetten ungeordnet, lose geschüttet enthalten sind. Anschließend wird die Stechhilfe, beispielsweise durch Abschrauben oder Abziehen einer Schutzkappe, für die Aufnahme der Lanzette vorbereitet, wobei der Lanzettenhalter der Stechhilfe freigelegt wird. Der Lanzettenhalter dient einerseits der Aufnahme der Lanzetten. Andererseits wird durch ihn die Lanzette beim eigentlichen Stechvorgang geführt. Die aus der Packung entnommene Lanzette wird manuell in den Lanzettenhalter der Stechhilfe eingeführt und dort fixiert. Dann muß die Schutzhülle, welche die Lanzettenspitze umgibt und sowohl diese als auch den Benutzer schützt, von der Lanzette manuell abgenommen werden. Anschließend wird die Stechhilfe mit ihrer Schutzkappe wieder verschlossen. Die Schutzkappe sorgt dafür, daß die Lanzette von außen nicht mehr zugänglich ist. Sie besitzt meist eine Öffnung, durch welche die Lanzettenspitze beim eigentlichen Stechvorgang austreten kann. Schließlich wird die Stechhilfe gespannt und steht für den Stechvorgang zur Gewinnung von Blut zur Verfügung.

Die Vielzahl der manuellen Bedienschritte bei herkömmlichen Lanzettensystemen (Lanzette und Stechhilfe) wird vom Benutzer als nachteilig empfunden und ist vor allem bei eingeschränkter Wahrnehmung im Zustand einer Hypoglykämie problematisch. Zudem wird der Benutzer nicht daran gehindert, eine einmal eingelegt Lanzette mehrfach zum Stechen und Blutgewinnen zu verwenden. Dies ist zum einen aus hygienische Gründen bedenklich, insbesondere wenn mehr als eine Person das Lanzettensystem benutzt, wie dies beispielsweise in Arztpraxen oder Krankenhäusern der Fall sein kann. Zum anderen führt die mehrmalige Benutzung der Lanzetten zu steigendem Schmerz für den Benutzer, denn da die Lanzetten für den einmaligen Gebrauch konzipiert sind, werden sie bei mehrmaligem Gebrauch schnell stumpf. Zudem besteht mit den Stechhilfen und Lanzetten des Standes der Technik die Gefahr, daß Stechhilfen mit nicht passenden Lanzetten benutzt werden, das heißt mit Lanzetten, die für einen speziellen Stechhilfentyp nicht geeignet sind und deshalb keine optimalen Stechergebnisse (Reproduzierbarkeit, Schmerzarmut, resultierende Blutmenge) erreicht werden, oder daß die Lanzetten unsachgemäß in die Stechhilfen eingelegt werden. Weiterhin kann sich ein Benutzer bei unsachgemäße Benutzung von Lanzetten und Stechhilfen unbeabsichtigt verletzen.

Es mangelt deshalb nicht an Versuchen, die angesprochenen Nachteile zu beseitigen. Aus US 5,514,152, US 5,152,775, WO-A 98/14125, US 3,030,959, US 4,794,926 und US 5,035,704 sind Stechhilfen bekannt, die mehrere Lanzetten in sich bevorraten und diese nacheinander einzeln für Stechvorgänge benutzen können. Nach dem Stechvorgang können die Lanzetten einzeln aus dem Gerät entfernt werden. Die oben angesprochenen Probleme der gebrauchten Lanzetten sind in diesen Systemen mit Lanzettenberrorratung jedoch ebensowenig gelöst wie bei Systemen, die manuell einzeln mit Lanzetten bestückt werden müssen.

Zusammenfassend ist festzustellen, daß alle Konzepte des Standes der Technik für Lanzetten und Lanzettensysteme, d. h. Stechhilfen oder Geräte, die Lanzetten in sich bevorraten können, den Nachteil aufweisen, daß die Sterilität, das heißt die Keimfreiheit, der unbenutzten Lanzettennadel, insbesondere deren Spitze, nicht bis unmittelbar vor dem Stechvorgang gewährleistet wird und eine sichere und hygienische Entsorgung der einmal benutzten Lanzette vollkommen dem Benutzer überlassen ist. Insbesondere für Systeme, in denen unbenutzte Lanzetten neben benutzten Lanzetten aufbewahrt werden sollen, also insbesondere bei Lanzettenmagazinen und entsprechenden Stechhilfen, die darauf verzichten, eine einmal benutzte Lanzette sofort auszuwerfen und statt dessen die benutzten Lanzetten bis zum Aufbrauchen des gesamten Lanzettenvorrats aufbewahren, stellt dies ein deutliches Problem dar.

Aufgabe der Erfindung ist es, die Nachteile des Standes der Technik zu beseitigen. Insbesondere ist es die Aufgabe der vorliegenden Erfindung, Lanzetten bereitzustellen, bei denen zumindest die Lazaxettennadelspitze im unbenutzten Zustand bis unmittelbar vor der Benutzung steril, das heißt keimfrei, gehalten wird und im benutzten Zustand hygienisch aufbewahrt werden kann. Idealerweise sollte diese Aufgabe gelöst werden, ohne daß der Benutzer für die hygienische Aufbewahrung separate Maßnahmen zu ergreifen hat. Zudem sollte der Benutzer vor unbeabsichtigter Verletzung mit der Lanzette, insbesondere der benutzten Lanzette geschützt sein.

Die Aufgabe wird durch den Gegenstand der Erfindung, wie er in den Patentansprüchen charakterisiert ist, gelöst.

Gegenstand der Erfindung ist eine Lanzette enthaltend eine Lanzettennadel mit einer Spitze und einen Lanzettenkörper, der die Lanzettennadel zumindest im Bereich der Spitze vollständig umgibt, wobei bei der erfindungsgemäßen Lanzette der Lanzettenkörper zumindest im Bereich der Spitze der Lanzettennadel aus einem elastischen Material besteht, in das die Spitze der Lanzettennadel vor ihrem Gebrauch ohne Verbleib eines Hohlraumes eingebettet ist.

Die erfindungsgemäßen Lanzetten sind für den einmaligen Gebrauch konzipiert und sollen daher auch als Einweg-Blutlanzetten oder Wegwerf-Blutlanzetten bezeichnet werden. Die Lanzette der Erfindung beinhaltet eine Nadel (Lanzettennadel) mit einer Spitze. Die Nadel ist in der Regel mehrere Millimeter (mm) bis wenige Zentimeter (cm) lang und weist eine längliche Gestalt auf. Typischerweise besitzen Nadeln eine zylindrische Gestalt, da diese Nadelform besonders gut herstellbar ist; es sind jedoch auch Nadelformen mit abweichender Formgebung möglich. Der Spitzenbereich der Nadel beinhaltet die Nadelspitze, die beim bestimmungsgemäßen Gebrauch der Lanzette in Gewebe eingestochen wird. Die Spitze der Lanzettennadel ist folglich der Teil der Lanzette, der mit der Haut des zu stechenden Individuums in Berührung kommt, diese ggf. verletzt und so den Ausfluß einer Körperflüssigkeit, insbesondere Blut oder interstitieller Flüssigkeit, verursacht.

Die Spitze der Lanzettennadel kann beispielsweise rotationssymmetrisch sein, wie dies im Allgemeinen bei Nähnadeln der Fall ist. Es hat sich jedoch als vorteilhaft herausgestellt, wenn man an der Nadelspitze einen oder mehrere Schliffe anbringt. Die hierbei entstehenden, zur Längsachse der Nadel geneigten, in einer Spitze zulaufenden Kanten dienen beim Einstich als scharfe Schneide und gestalten den Einstichvorgang schmerzärmer, als dies mit rotationssymmetrischen Nadeln der Fall ist.

Die Lanzettennadel der erfindungsgemäßen Lanzette ist aus einem Material gefertigt, das ausreichend hart ist, um eine mechanische Beanspruchung während des Einstichvorgangs, der Bearbeitungsschritte oder eventuell sonstige auftretende Beanspruchungen ohne Deformation zu überstehen. Weiterhin muß das Material so beschaffen sein, daß während des Einstichvorgangs keine Partikel abbrechen oder sich ablösen. Schließlich muß das Nadelmaterial auch so bearbeitbar sein, daß die Nadelspitze ausreichend spitz und die Kanten der Nadelspitze gegebenenfalls ausreichend scharf geschliffen werden können. Gut geeignete Materialien für die Lanzettennadel sind vor allem Metalle und von diesen insbesondere Edelstähle. Es sind jedoch auch Nadeln aus Keramik oder Kunststoffen denkbar. Edelstahlnadeln sind besonders bevorzugt

Erfindungsgemäß ist zumindest die Spitze der Lanzettennadel der erfindungsgemäßen Lanzette von einem Kunststoffkörper, der im folgenden als Lanzettenkörper bezeichnet werden soll, umgeben. Wesentlich ist dabei, daß der Lanzettenkörper im Bereich der Spitze der Lanzettennadel aus einem elastischen Material besteht Zumindest die Spitze der Lanzettennadel ist vor ihrem Gebrauch von diesem elastischen Material allseitig vollständig umgeben, das heißt in dieses eingebettet, und so von der Umgebung abgeschlossen ohne Verbleib eines Hohlraumes. Das elastische Material des Lanzettenkörpers, welches in unterschiedlichen Aus-Rihrungsformen den Lanzettenkörper vollständig oder nur teilweise bilden kann, zeichnet sich dadurch aus, daß es weich, verformbar und von der Lanzettennadel mit ihrer Spitze durchstoßbar ist, ohne die Spitze zu verletzen. Beim Stechvorgang wird die Lanzettennadel entlang ihrer Längsachse relativ zum Lanzettenkörper bewegt und tritt mit ihrer Spitze aus dem Lanzettenkörper aus, um so zur Blutgewinnung in die Haut des zu untersuchenden Individuums einstechen zu können. Eine wichtige Eigenschaft ist ferner, daß sich das elastische Material gegebenenfalls beim Zurückziehen der Lanzettennadel in den Lanzettenkörper wieder dicht um die Spitze der Lanzettennadel verschließt. Nach dem Stechvorgang kann die Lanzettennadel in einer bevorzugten Ausführungsform in Umkehrung der Bewegung beim Stechvorgang in ihre Ausgangslage relativ zum Lanzettenkörper gebracht werden, in der die Spitze wieder allseitig vollständig vom elastischen Material des Lanzettenkörpers umschlossen ist.

Das elastische Material des Lanzettenkörpers, welches die Spitze der Lanzettennadel vollständig umschließt, gewährleistet die Sterilität der Lanzettennadelspitze vor deren Benutzung, vorzugsweise bis unmittelbar vor deren Benutzung, und sorgt gegebenenfalls für ein hygienisches Umschließen der Lanzettennadelspitze nach deren Benutzung. Das elastische Material ist folglich keimdicht für das Eindringen oder Entweichen von Keimen, je nach dem, ob die Lanzettennadel unbenutzt oder benutzt ist. Zudem stellt das elastische Material einen mechanischen Schutz für die Lanzettennadelspitze dar und verhindert so auch ein unbeabsichtigtes Verletzen an der Lanzettennadelspitze.

Als elastisches Material für den Lanzettenkörper der vorliegenden Erfindung haben sich Gummi, Kautschuk, Silikon, Elastomere und insbesondere thermoplastische Elastomere als geeignet herausgestellt. Diese weisen die für die vorliegende Erfindung wesentlichen Eigenschaften auf: sie sind weich, verformbar, von der Lanzettennadel zu durchstoßen, ohne die Spitze zu verletzen, und schließen sich dicht um die benutzte Lanzettennadelspitze. Des weiteren können Sie für Spritzgußprozesse verwendet werden, die eine Massenfertigung von Lanzetten in großen Stückzahlen ermöglichen.

Thermoplastische Elastomere, die auch Elastoplaste oder Thermolaste oder thermoplastische Kautschuke genannt werden, besitzen im Idealfall eine Kombination der Gebrauchseigenschaften von Elastomeren und den Verarbeitungseigenschaften von Thermoplasten. Thermoplastische Elastomere sind beispielsweise Styrol-Oligoblock-Copolymere (sogenannte TPE-S), thermoplastische Polyolefine (TPE-O), thermoplastische Polyurethane (TPE-U), thermoplastische Copolyester (TPE-E) und thermoplastische Copolyamide (TPE-A). Insbesondere haben sich beispielsweise thermoplastische Elastomere auf der Basis von Styrol-Ethylen-Butylen-Styrol-Polymeren (SEBS-Polymere, z. B. Evoprene^{®} von Evode Plastics oder Thermolast K von Gummiwerk Kraiburg GmbH) als geeignet erwiesen.

Während des Stechvorgangs wird die Lanzettennadel relativ zum Lanzettenkörper bewegt. Letzterer wird dabei vorzugsweise von der Stechhilfe oder dem Stechgerät in seiner Position fixiert. Die Lanzettennadel kann zum Zwecke ihres Antriebs besonders ausgeformt sein, beispielsweise einen Nadelkopf an dem der Spitze entgegengesetzten Ende besitzen, oder zusätzlich zum Lanzettenkörper, der die Spitze umschließt, einen weiteren Lanzettenkörper aufweisen, der von einem Antriebselement der Stechhilfe ergriffen werden kann. Die Ausformung der Nadel oder der zusätzliche Lanzettenkörper können in geeigneter Weise mit einer entsprechenden Antriebsvorrichtung im Stechgerät (Stechhilfe) wechselwirken.

Zur Erzielung des Vorteils, daß die Lanzettennadelspitze vor Gebrauch hygienisch vom elastischen Material des Lanzettenkörpers umschlossen ist, und nach ihrem Gebrauch ebenfalls hygienisch vom elastischen Material umgeben ist, ist es natürlich notwendig, die Lanzettennadel nach ihrem Gebrauch, d. h. nach dem Stechvorgang, weitgehend in ihre ursprüngliche relative Position bezüglich des Lanzettenkörpers enthaltend das elastische Material zu bringen. Dies kann durch geeignete Wechselwirkung mit einer entsprechend angepaßten Stechhilfe erreicht werden. Wichtig ist dabei nur, daß die Lanzettennadelspitze nach ihrer Benutzung wieder vom elastischen Material des Lanzettenkörpers umschlossen ist und so ein unbeabsichtigtes Verletzen an der Nadelspitze verhindert wird.

Zur Erhöhung der Stabilität des elastischen Materials ist es möglich, dieses mit einem steifen Material, beispielsweise einem steifen Kunststaffrnaterial, zu verbinden. Das elastische Material kann dabei beispielsweise auf seiner Außenseite, die nicht mit der Spitze der Lanzettennadel in Berührung kommt, mit einer Schicht eines steifen Materials, beispielsweise eines steifen Kunststoffs, stabilisiert sein. Es ist auch möglich, den Lanzettenkörper nur im Bereich der Lanzettennadelspitze aus einem elastischen Material zu fertigen, im übrigen den Lanzettenkörper jedoch aus herkömmlichen, steifen Kunststoffen zu fertigen. Dabei können das elastische Material und das steife Material miteinander verklebt sein oder durch einen Spritzgußprozeß, beispielsweise einen Zweikomponenten-Spritzgußprozeß, miteinander verbunden sein. Das steife Material des Lanzettenkörpers sorgt dabei für eine mechanische Stabilisierung des elastischen Materials während des Stechvorgangs und erleichtert die Fixierung des elastischen Teils des Lanzettenkörpers während des Stechvorgangs durch die Stechhilfe.

Alternativ ist eine Ausführungsform denkbar mit einer Lanzette enthaltend eine Lanzettennadel mit einer Spitze und einen Hohlkörper, der zumindest die Spitze der Lanzettennadel umgibt, wobei bei der erfindungsgemäßen Lanzette die Lanzettennadel im Bereich ihrer Spitze im Hohlkörper beweglich ist und der Hohlkörper zumindest teilweise aus einem elastischen Material besteht, das von der Spitze der Lanzettennadel beim Stechvorgang durchstoßen werden kann und das sich gegebenenfalls nach dem Zurückziehen der Spitze der Lanzettennadel in den Hohlkörper wieder verschließt.

Während bei der weiter oben beschriebenen Lanzette gemäß dem Gegenstand der Erfindung die Lanzettennadel zur Gewährung der Sterilität vor Benutzung und der hygienischen Abgeschirmtheit nach der Benutzung im Bereich ihrer Spitze allseitig vollständig und somit ohne verbleibenden Hohlraum um die Spitze von einem elastischen Material umgeben ist, welches die Lanzettennadelspitze in sich einbettet, ist bei dem jetzt beschriebenen zweiten Gegenstand die Spitze der Lanzettennadel von einem allseits geschlossenen Hohlkörper umgeben. Vorteilhafterweise ist dieser Hohlkörper in den Bereichen, die nicht mit der Spitze der Lanzettennadel in Berührung kommen, aus einem steifen, vorzugsweise spritzgußfähigen Material gefertigt. Hierbei besteht der Hohlkörper in dem Bereich, in dem er beim Stechvorgang von der Lanzettennadelspitze durchstoßen wird, aus einem elastischen Material.

Beim Stechvorgang wird die Lanzettennadel relativ zum Hohlkörper, der den Lanzettenkörper darstellt, bewegt. Halterung und Antrieb der Lanzettennadel und Fixierung des Lanzettenkörpers können, wie oben beschrieben, durch geeignete konstruktive Maßnahmen in der Stechhilfe realisiert werden.

Das elastische Material, welches einen Teil des hohlen Lanzettenkörpers ausmacht, wird beim Stechvorgang von der Lanzettennadelspitze durchstoßen und verschließt sich gegebenenfalls nach dem Zurückziehen der Lanzettennadelspitze in den Hohlkörper wieder und dichtet so den Hohlkörper ab. Die Lanzettennadelspitze ist somit bis unmittelbar vor Gebrauch steril im Hohlkörper versiegelt und wird nach Gebrauch hygienisch in ihm eingeschlossen.

Die Lanzette dieses Erfindungsgegenstands kann ebenso wie die Lanzette des oben beschriebenen Gegenstandes neben dem Lanzettenkörper, der die Spitze der Lanzettennadel umschließt, einen weiteren Lanzettenkörper aufweisen, der im Zusammenspiel mit geeigneten Elementen einer Stechhilfe während des Stechvorgangs mit diesen wechselwirkt. Ebenso kann die Lanzettennadel speziell geformt sein, beispielsweise einen Kopf an dem der Spitze entgegengesetzten Ende besitzen.

Für die Eigenschaften des elastischen Materials und die Verbindung des elastischen Material mit dem steifen Material des Lanzettenkörpers gilt entsprechend das oben bezüglich des ersten Gegenstandes der Erfindung Gesagte.

Zusätzlich ist vorteilhafterweise Gegenstand der Erfindung ein Lanzettensatz, der mindestens zwei der erfindungsgemäßen Lanzetten enthält, die miteinander verbunden sind. Dies ist der dritte Gegenstand der Erfindung.

Die Lanzetten des Lanzettensatzes gemäß der vorliegenden Erfindung sind von der Art, daß die Lanzettennadelspitze von einem elastischen Material ohne Verbleib eines Hohlraumes um die Spitze allseitig vollständig umschlossen oder eingebettet ist Die einzelnen Lanzetten, die jeweils wiederum aus mindestens einer Lanxettennadel und einem Lanzettenkörper bestehen, sind im erfindungsgemäßen Lanzettensatz miteinander verbunden. Vorteilhafterweise erfolgt die Verbindung über die Lanxettenkörper. Bevorzugt werden identische Lanzetten miteinander verbunden.

Die Lanzetten können im Lanzettensatz entweder über dünne Stege oder Brücken miteinander verbunden sein oder auf einem Trägerband, beispielsweise aus Papier oder Kunststoff, befestigt sein. Vorzugsweise geschieht die Verbindung der Lanzetten derart, daß die einzelnen Lanzettennadeln der einzelnen Lanzetten des Lanzettensatzes in einem durchgehenden Stück des elastischen Materials eingeschlossen sind. Das elastische Material kann in diesem Fall die Form eines elastischen Bandes aufweisen. Das elastische Band als Verbindungsform für mehrere, vorzugsweise mehrere gleiche Lanzetten ist vorzugsweise für die Lanzetten gemäß des ersten Gegenstandes der Erfindung, das heißt Lanzetten, bei denen die Lanzettennadelspitze vollständig in das elastische Material eingebettet ist, geeignet. Es ist jedoch auch möglich, ein elastisches Bandmaterial als Verbindungsmaterial für Lanzetten gemäß des zweiten Gegenstandes der Erfindung, das heißt Lanzetten mit einem Hohlkörper um die Lanzettennadelspitze, einzusetzen.

Außerdem ist Gegenstand der Erfindung ein Lanzettenmagazin mit Kammern zur Aufbewahrung von Lanzetten. Das erfindungsgemäße Magazin enthält mindestens zwei Lanzetten, die jeweils eine Lanzettennadel mit einer Spitze enthalten und die jeweils in einzelnen, vozzeinander unabhängigen Kammern des Lanzettenmagazins untergebracht sind Dabei weist jede Kammer zumindest eine Öffnung für den Austritt der Spitze der Lanzettennadel auf. Das Lanzettenmagazin der vorliegenden Erfindung zeichnet sich dadurch aus, daß die besagte Öffnung der Kammer von einem elastischen Material verschlossen ist Das elastische Material wird von der Spitze der Lanzettennadel beim Stechvorgang durchstoßen und verschließt sich nach dem Zurückziehen der Spitze der Lanzettennadel in die Kammer wieder.

Vorteilhafterweise dient das Lanzettenmagazin der gemeinsamen Aufbewahrung unbenutzter Lanzetten (Kassettierung) und gegebenenfalls auch der Aufbewahrung einmal benutzter Lanzetten (Wiederkassettierung). Anders als beim Lanzettensatz sind die einzelnen Lanzetten im Falle des Lanzettenmagazins nicht direkt miteinander verbunden, sondern befinden sich in einzelnen Kammern des Magazins. In diesen liegen sie unabhängig voneinander vor. Im Magazin sind die Kammern im wesentlichen regelmäßig geometrisch angeordnet, wobei benachbarte Kammern zumindest eine, vorzugsweise zwei gemeinsame Wa(e)nd(e) aufweisen.

Die einzelnen Kammern des Magazins besitzen zumindest eine Austrittsöffnung für die Spitze der Lanzettennadel. Diese Austrittsöffnung ist erfindungsgemäß durch ein elastisches Material verschlossen, das die weiter oben genannten Eigenschaften aufweist, die im Zusammenhang mit dem ersten Gegenstand der Erfindung erörtert wurden. Während des Stechvorgangs kann die Spitze der Lanzettennadel durch das elastische Material hindurchdringen. Das elastische Material schließt sich gegebenenfalls wieder beim Zurückziehen der Lanzettennadelspitze in die Kammer des Lanzettenmagazins. Die Kammer ist dann wieder dicht verschlossen.

Durch geeignete konstruktive Maßnahmen kann die Kammer des Lanzettenmagazins so abgedichtet werden, daß die Lanzettennadel vor Benutzung im Bereich ihrer Spitze steril ist und gegebenenfalls nach Benutzung hygienisch von der Umwelt abgeschirmt ist. Beispielsweise kann die Lanzette einen Lanzettenkörper aufweisen, der durch geeignete Formgebung mit entsprechenden Formen der Innenwand der Kammer des Lanzettenmagazins wechselwirkt, um so ein Abdichten zu ermöglichen. Erfindungswesentlich ist nur, daß die Öffnung der Kammer, durch die die Lanzettennadelspitze während des Stechvorgangs hindurchtritt, von dem elastischen Material verschlossen ist.

Die Anordnung der einzelnen Kammern im Lanzettenmagazin kann beliebig sein. Beispielsweise können eine Mehrzahl von Lanzettenkammern nebeneinander angeordnet sein und so ein im Wesentlichen quader- oder riegelförmiges Magazin ergeben. Es ist jedoch auch denkbar, daß die Kammern symmetrisch um eine zentrale Achse angeordnet sind, so daß sich ein Lanzettenmagazin in Form einer Trommel (ähnlich der Trommel eines Trommelrevolvers) ergibt. Beliebige andere Anordnungen sind jedoch auch denkbar und möglich.

Schließlich ist Gegenstand der Erfindung die Verwendung eines elastischen Materials als Bestandteil einer Lanzette oder eines Lanzettenmagazins, wobei das elastische Material dazu dient, die Sterilität zumindest der Spitze einer Lanzettennadel im unbenutzten Zustand zu erhalten. In einer bevorzugten Ausführungsform kann das elestische Material auch dazu verwendet werden, ein hygienisches Abschirmen zumindest der Spitze einer Lanzettennadel im benutzten Zustand zu erreichen.

Die erfindungsgemäße Verwendung eines elastischen Materials zum Abschirmen der Spitze der Lanzettennadel erlaubt es, die Sterilität einer unbenutzten Lanzettennadelspitze zu gewährleisten und gegebenenfalls die benutzte Lanzettennadelspitze hygienisch abzuschirmen.

Die Sterilität der Lanzettennadelspitze im unbenutzten Zustand ist durch geeignete Maßnahmen, wie beispielsweise Gamma-Bestrahlung, herzustellen. Einmal sterilisiert bleiben die Lanzettennadelspitzen durch die entsprechenden Lanzettenkörper oder ein entsprechendes Lanzettenmagazin, die unter anderem ein elastisches Material beinhalten, erhalten. Anders als im Stand der Technik, wo bislang keine elastischen Materialien zur Abschirmung von Lanzettennadelspitzen beschrieben sind, ermöglicht die Verwendung des elastischen Materials gemäß der vorliegenden Erfindung jedoch auch die hygienische Abschirmung der benutzten Lanzettennadelspitze. Durch die Verwendung des elastischen Materials wird ein eventuell kurzzeitig vorhandener Kanal, durch den die Lanzettennadel zum Zwecke des Stechens hindurchdringen kann, nach dem Zurückziehen der Lanzettennadel, das heißt nach vollzogenem Stechvorgang, wieder verschlossen. Eventuell nach dem Stechvorgang an der Lanzettennadelspitze anhaftende Kontaminationen, insbesondere Keime und infektiöses Material, können somit nicht an die Außenwelt gelangen. Dies ist von besonderem Vorteil für Einweglanzetten, die einzeln nach ihrer Benutzung entsorgt werden. Von ganz hervorragender Bedeutung ist diese Eigenschaft jedoch für Lanzettensätze und Lanzettenmagazine, bei denen neben unbenutzten auch benutzte Lanzetten gelagert werden, und die dann als Ganzes entsorgt werden können.

Die Erfindung weist die folgenden Vorteile auf:
- Die Spitze der Lanzettennadel ist bei allen Ausführungsformen im unbenutzten Zustand keimdicht abgeschirmt, das heißt, Keime können bis unmittelbar vor Benutzung der Lanzette nicht an die Lanzettennadelspitze vordringen. Nach geeigneter Sterilisierung bleiben die Lanzettenspitzen über lange Zeit steril.
- Die Spitze der Lanzettennadel kann bei allen Ausführungsformen im benutzten Zustand hygienisch abgeschirmt werden. Eine unbeabsichtigte Kontamination der Umgebung (Benutzer, Gegenstände, weitere Lanzetten) wird weitgehend ausgeschlossen.
- Der Benutzer der erfindungsgemäßen Lanzetten ist vor unbeabsichtigter Verletzung an einer benutzten Lanzettennadel geschützt. Gleiches gilt natürlich für andere Personen als den eigentlichen Benutzer.
- Die erfindungsgemäßen Lanzetten und Lanzettensätze sind kostengünstig in großen Stückzahlen mit herkömmlichen Spritzgußverfahren herzustellen.
- Die erfindungsgemäßen Lanzetten und Lanzettensätze sind weitgehend miniaturisierbar und eignen sich deshalb für den Einsatz in kompakten, automatisierten Systemen.

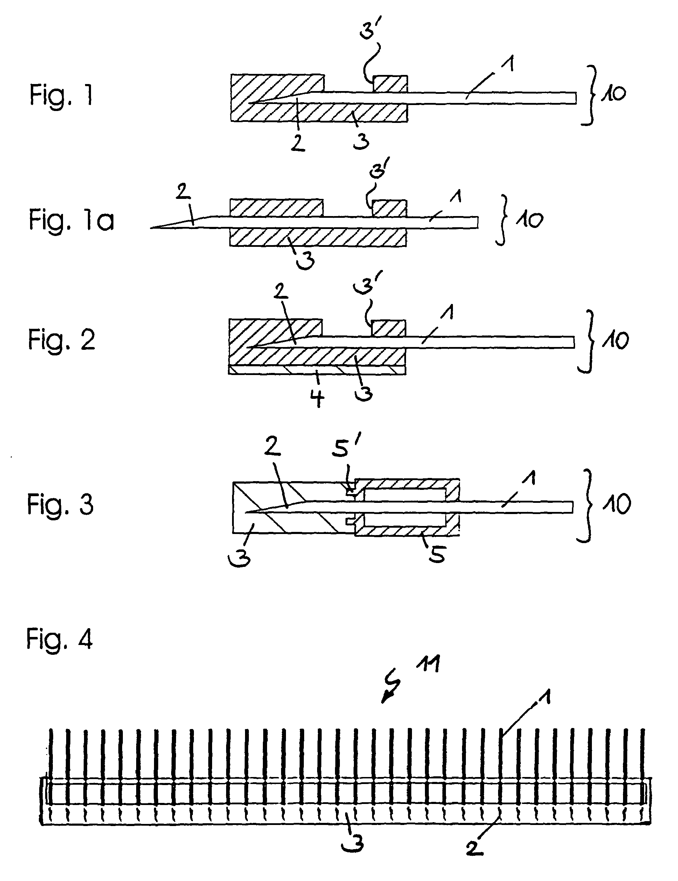

Die Erfindung wird durch die nachfolgenden Figuren 1 bis 6 näher erläutert.

Die Ziffern in den Figuren bedeuten:
- 1: Lanzettennadel
- 2: Spitze der Lanzettennadel
- 3: Lanzettenkörper aus elastischem Material
- 3': Kante des Lanzettenkörpers 3
- 4: Stabilisierungsschicht
- 5: Lanzettenkörper aus starrem Material
- 5': Vorsprung des Lanzettenkörpers
- 6: Verschluß aus elastischem Material
- 7: Lanzettenkörper
- 8: Kammerwand
- 9: Lanzettenkörper
- 10: Lanzette
- 11: Lanzettensatz
- 12: Lanzette (10) in Kammer

In Figur 1 ist schematisch ein Längsschnitt durch eine bevorzugte Ausführungsform einer erfindungsgemäßen Lanzette abgebildet.

In Figur 2 ist schematisch ein Längsschnitt durch eine alternative, ebenfalls bevorzugte Ausführungsform einer erfindungsgemäßen Lanzette abgebildet.

In Figur 3 ist schematisch ein Längsschnitt durch eine weitere Alternative einer bevorzugten Ausführungsform der erfindungsgemäßen Lanzette abgebildet.

Figur 4 zeigt eine schematische Aufsicht auf eine bevorzugte Ausführungsform eines erfindungsgemäßen Lanzettensatzes.

In Figur 5 ist ein schematischer Längsschnitt durch eine Kammer eines bevorzugten erfindungsgemäßen Lanzettenmagazins einschließlich einer darin befindlichen Lanzette abgebildet.

Figur 6 zeigt eine weitere Lanzette in einer schematischen Schnittdarstellung.

Die in Figur 1 dargestellte bevorzugte Ausführungsform der erfindungsgemäßen Lanzette (10) enthält eine Lanzettennadel (1), die im Bereich ihrer Spitze (2) von einem Lanzettenkörper (3), der aus einem elastischen Material gefertigt ist, umgeben ist. Die Spitze (2) der Lanzettennadel (1) ist vollständig vom elastischen Material des Lanzettenkörpers (3) umgeben. Außerhalb des Bereichs der Spitze (2) der Lanzettennadel (1) weist der Lanzettenkörpers (3) eine Aussparung auf, die dazu führt, daß der Lanzettenkörper (3) zumindest auf einer Seite eine Kante (3') besitzt, mit der entsprechende Greif oder Haltevorrichtungen in einer Stechhilfe wechselwirken können, um den Lanzettenkörper (3) festzuhalten. Das Festhalten des Lanzettenkörpers (3) ist erforderlich, da während des Stechvorgangs die Lanzettennadel (1) relativ zum Lanzettenkörper (3) bewegt werden muß und durch diesen im Bereich der Spitze (2) der Lanzettennadel (1) hindurchtreten können muß. Figur 1 a zeigt die Lanzette (10) am Ende der Vorwärtsbewegung des Stechvorgangs.

Die Aussparung im Lanzettenközper (3) dient zudem der Reduzierung der Reibungskräfte zwischen Lanzettennadel (1) und Lanzettenkörper (3) während der Stechbewegung.

Der Querschnitt der Lanzettennadel (1) und des Lanzettenkörpers (3) senkrecht zur Längsachse der Nadel weist bevorzugt eine runde Form auf. Es ist jedoch auch möglich, daß der Querschnitt eine beliebige andere Form aufweist, beispielsweise quadratisch oder rechteckig ist. Es ist auch nicht erforderlich, daß die Lanzettennadel (1) und der Lanzettenkörper (3) im Querschnitt die gleiche Form aufweisen. Beispielsweise kann die Lanzettennadel (1) einen im wesentlichen runden Querschnitt und der Lanzettenkörper (3) einen rechteckigen Querschnitt besitzen.

In Figur 2 ist eine weitere ebenfalls bevorzugte Ausführungsform einer erfindungsgemäßen Lanzette (10) schematisch im Längsschnitt dargestellt. Die Lanzette (10) besteht im wesentlichen aus den selben Elementen, wie die in Figur 1 dargestellte Lanzette (10). Abweichend von der in Figur 1 dargestellten Lanzette (10) ist auf einer Seite des Lanzettenkörpers (3), der aus elastischem Material besteht, eine Stabilisierungsschicht (4) aufgebracht. Diese Stabilisierungsschicht (4) kann mit dem Lanzettenkörper (3) verklebt sein. Es ist jedoch bevorzugt, daß die Stabilisierungsschicht (4) zusammen mit dem Lanzettenkörper (3) in einem Spritzgußverfahren mit der Lanzettennadel (1) verbunden wird.

Die Stabilisierungsschicht (4) dient dazu, eine Verformung des elastischen Lanzettenkörpers (3) während des Stechvorgangs zu verhindern. Insbesondere soll eine Dehnung des elastischen Lanzettenkörpers (3) verhindert werden.

In Figur 3 ist eine weitere bevorzugte Ausführungsform der erfindungsgemäßen Lanzette (10) in einem schematischen Längsschnitt dargestellt. Der Lanzettenkörper (3, 5) der Lanzette (10) besteht in diesem bevorzugten Fall aus zwei unterschiedlichen Komponenten. Die Spitze (2) der Lanzettennadel (1) ist von einem Lanzettenkörper (3) aus einem elastischen Material umschlossen. Abseits des Spitzenbereichs (2) der Lanzettennadel (1) ist die Lanzettennadel (1) mit einem Lanzettenkörper (5) aus einem starren Material verbunden. Der Lanzettenkörper (5) aus starrem Material ist wiederum mit dem Lanzettenkörper (3) aus elastischem Material verbunden. Um die Verbindungsoberfläche zwischen elastischem Lanzettenkörpermaterial (3) und starrem Lanzettenkörpermaterial (5) zu vergrößern, kann das starre Lanzettenkörpermaterial (5) Vorsprünge 5' aufweisen. Die Verbindung zwischen starrem und elastischem Material (3, 5) des Lanzettenkörpers kann wiederum durch Kleben oder Zweikomponenten-Spritzguß erfolgen.

Während im Fall der Ausführungsformen gemäß Figuren 1 und 2 ein Halten des Lanzettenkörpers (3) während des Stechvorgangs durch unmittelbares Einwirken auf das elastische Material des Lanzettenkörpers (3) erfolgt, wird im Fall der Ausführungsform gemäß Figur 3 der Lanzettenkörper (5), welcher aus einem starren Material besteht, beim Stechvorgang gehalten. Die Lanzettennadel (1) wird dabei entlang ihrer Längsachse bewegt. Sie durchdringt dabei die Oberfläche des elastischen Lanzettenkörpers (3). Nach dem Stechvorgang wird die Lanzettennadel (1) entsprechend zurückgeführt, so daß sich der elastische Lanzettenkörper (3) wieder um die Spitze (2) der Lanzettennadel (1) legen kann. Der Lanzettenkörper (3, 5) wird während des Stechvorgangs in seiner Position fixiert.

In Figur 4 ist eine schematische Aufsicht auf einen Lanzettensatz (11) abgebildet. Eine Vielzahl von Lanzettennadeln (1) mit einer Spitze (2) sind in dieser Ausführungsform des Lanzettensatzes (11) in einen einzigen bandartigen Lanzettenkörper (3) aus elastischem Material eingebettet. Der Lanzettensatz (11) ist zur Verwendung in einem automatisierten System geeignet, in dem eine Vielzahl von Lanzetten bevorratet und einzeln für Stechvorgänge benutzt werden können.

Ein Längsschnitt durch die Längsachse einer einzelnen Lanzette des Lanzettensatzes (11) entspräche im wesentlichen der Darstellung der Lanzette (10) in Figur 1 oder 2. Der Lanzettensatz (11) kann - analog der Lanzette aus Figur 2 - eine Stabilisierungsschicht (4) (in Figur 4 nicht gezeigt) aufweisen. Diese stabilisiert den Lanzettensatz (11) als ganzen und die einzelnen, in ihm enthaltenen Lanzetten nicht nur während des Stechvorgangs, sondern verleiht ihm gleichzeitig eine mechanische Festigkeit, die beispielsweise für die automatisierte Handhabung des Lanzettensatzes (11) in einem mechanischen System zur Bevorratung und Verwendung der Lanzetten vorteilhaft sein kann.

Da der Lanzettenkörper (3) des Lanzettensatzes (11) aus einem elastischen Material besteht, ist es möglich, den gesamten Lanzettensatz (11) kompakt zu verpacken, beispielsweise durch spiralförmiges Zusammenrollen um die Längsachse der ersten Lanzettennadel (2) im Lanzettensatz (11).

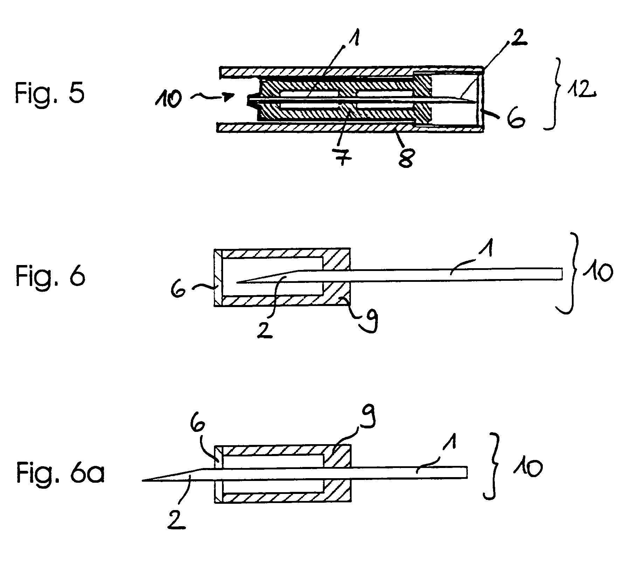

In Figur 5 ist ein schematischer Längsschnitt durch eine Kammer eines Lanzettenmagazins, in der eine Lanzette (10) enthalten ist, abgebildet. Im Lanzettenmagazin können die einzelnen Kammern beliebig angeordnet sein. Beispielsweise können die Kammern neben- oder hintereinander angeordnet sein und so ein im wesentlichen quaderförmiges Magazin bilden oder aber um eine zentrale Achse radial angeordnet sein und so im wesentlichen ein zylindrisches oder trommelförmiges Magazin bilden. Die Lanzette (10) wird in der Kammer von den Kammerwänden (8) umschlossen. In der gezeigten Ausführungsform der Figur 5 weisen der Lanzettenkörper (7), der die Lanzettennadel (1) umgibt, und die Kammerwand (8) eine aneinander angepaßte komplementäre Form auf, so daß sich im Bereich der Spitze (2) der Lanzettennadel (1) ein geschlossener Hohlraum befindet. Neben der Kammerwand (8) und dem Lanzettenkörper wird der Hohlraum durch einen Verschluß (6) aus elastischem Material abgeschlossen. Der Verschluß (6) kann beim Stechvorgang durch die Spitze (2) der Lanzette (10) durchdrungen werden und verschließt sich nach dem Zurückziehen der Lanzette (10) in die Kammer des Magazins, ähnlich dem Septum einer Glasampulle, die beispielsweise zum Aufbewahren von Vakzinen verwendet werden. Der Verschluß (6) aus elastischem Material gewährleistet somit eine hygienische Lagerung und Entsorgung einer bereits benutzten Lanzette (10).

Eine analoge "Septumfunktion" erfüllt der Verschluß (6) des Lanzettenkörpers (9) in der Lanzette (10) gemäß Figur 6. Diese Lanzette (10) besteht aus einer Lanzettennadel (1) mit einer Spitze (2), die sich in einem geschlossenen Hohlraum befindet, der aus dem Lanzettenkörper (9), welcher aus einem starren Material gefertigt ist, und dem Verschluß (6) aus elastischem Material gebildet wird. Beim Stechvorgang gleitet die Lanzettennadel (1) mit ihrer Spitze (2) voran entlang ihrer Längsachse, während der Lanzettenkörper (9) durch geeignete Mittel in einer Stechhilfe unbeweglich gehalten wird. Dabei durchdringt die Spitze (2) der Lanzettennadel (1) den Verschluß (6) aus elastischem Material. Figur 6 a zeigt die Lanzette (10) am Ende der Vorwärtsbewegung des Stechvorgangs.

Die elastische Materialeigenschaft des Verschlusses (6) sorgt dafür, daß nach Zurückziehen der Lanzettennadel (1) nach dem Stechvorgang und insbesondere nach dem Zurückziehen der Spitze (2) der Lanzettennadel (1) in den Hohlraum des Lanzettenkörpers (9), eben jener Hohlraum wieder verschlossen ist.

## Patentansprüche

1. Lanzette enthaltend
eine Lanzettennadel mit einer Spitze und
einen Lanzettenkörper, der die Lanzettennadel zumindest im Bereich der Spitze vollständig umgibt, wobei die Spitze relativ zum Lanzettenkörper beweglich ist und aus dem Lanzettenkörper austreten kann und der Lanzettenkörper zumindest im Bereich der Spitze der Lanzettennadel aus einem elastischen Material besteht, **dadurch gekennzeichnet, dass** die Spitze der Lanzettennadel vor ihrem Gebrauch im elastischen Material ohne Verbleib eines Hohlraumes eingebettet ist

2. Lanzette gemäß einem der Ansprüche 1, **dadurch gekennzeichnet, dass** das elastische Material ein thermoplastisches Elastomer ist.

3. Lanzette gemäß einem der Ansprüche 1 bis 2, **dadurch gekennzeichnet, dass** das elastische Material mit einem steifen Material stabilisiert ist.

4. Lanzettensatz enthaltend mindestens zwei Lanzetten gemäß einem der Ansprüche 1 bis 3, die miteinander verbunden sind.

5. Lanzettensatz gemäß Anspruch 4, **dadurch gekennzeichnet, dass** die Lanzetten über das elastische Material miteinander verbunden sind.

6. Lanzettenmagazin enthaltend mindestens zwei Lanzetten, die jeweils eine Lanzettennadel mit einer Spitze enthalten und die jeweils in einzelnen, voneinander unabhängigen Kammern des Lanzettenmagazins untergebracht sind, wobei jede Kammer zumindest eine Öffnung für den Austritt der Spitze der Lanzettennadel aufweist, wobei die besagte Öffnung der Kammer von einem elastischen Material verschlossen ist, so dass das elastische Material von der Spitze der Lanzettennadel beim Stechvorgang durchstoßen werden kann, **dadurch gekennzeichnet, dass** sich das elastische Material nach dem Zurückziehen der Spitze der Lanzettennadel in die Kammer wieder dicht verschließt.

7. Lanzettenmagazin gemäß Anspruch 6, **dadurch gekennzeichnet, dass** das elastische Material ein thermoplastisches Elastomer ist

8. Verwendung eines elastischen Materials als Bestandteil einer Lanzette gemäß einer der Ansprüche 1-5 zum Erhalten der Sterilität zumindest der Spitze einer Lanzettennadel im unbenutzten Zustand.

9. Verwendung eines elastischen Materials als Bestandteil einer Lanzette gemäß einer der Ansprüche 6-7 zum Erhalten der Sterilität zumindest der Spitze einer Lanzettennadel im unbenutzten Zustand.

10. Verwendung gemäß Anspruch 8 oder 9, wobei das elastische Material zusätzlich zum hygienischen Abschirmen zumindest der Spitze einer Lanzettennadel im benutzten Zustand verwendet wird.

11. Verwendung gemäß Anspruch 8 oder 9, **dadurch gekennzeichnet, dass** das elastische Material ein thermoplastisches Elastomer ist.

## Claims

1. Lancet containing
a lancet needle with a tip and
a lancet body which completely surrounds the lancet needle at least in the area of the tip, the tip being able to move relative to the lancet body and able to protrude from the lancet body and the lancet body consisting of an elastic material at least in the area of the tip of the lancet needle, **characterized in that** before its use, the tip of the lancet needle is embedded in the elastic material without a hollow space remaining.

2. Lancet according to claim 1, **characterized in that** the elastic material is a thermoplastic elastomer.

3. Lancet according to one of the claims 1 to 2, **characterized in that** the elastic material is stabilized with a stiff material.

4. Lancet set comprising at least two lancets according to one of the claims 1 to 3 which are connected together.

5. Lancet set according to claim 4, **characterized in that** the lancets are connected together by the elastic material.

6. Lancet magazine containing at least two lancets which each comprise a lancet needle with a tip and which are each accommodated in individual chambers of the lancet magazine that are independent of one another, each chamber having at least one opening for the tip of the lancet needle to emerge, the said opening of the chamber being closed by an elastic material such that the elastic material can be pierced by the tip of the lancet needle during the lancing process, **characterized in that** the elastic material tightly reseals after the tip of the lancet needle has been retracted into the chamber.

7. Lancet magazine according to claim 6, **characterized in that** the elastic material is a thermoplastic elastomer.

8. Use of an elastic material as a component of a lancet according to one of the claims 1 to 5 to preserve the sterility of at least the tip of a lancet needle in the unused state.

9. Use of an elastic material as a component of a lancet according to one of the claims 6 to 7 to preserve the sterility of at least the tip of a lancet needle in the unused state.

10. Use according to claim 8 or 9, **characterized in that** the elastic material is additionally used to hygienically shield at least the tip of a lancet needle in the used state.

11. Use according to claim 8 or 9, **characterized in that** the elastic material is a thermoplastic elastomer.

## Revendications

1. Lancette contenant
une aiguille de lancette comprenant une pointe et
un corps de lancette qui entoure complètement l'aiguille de lancette au moins dans la zone de la pointe, la pointe étant mobile par rapport au corps de lancette et étant à même de sortir du corps de lancette, le corps de lancette étant constitué d'une matière élastique, au moins dans la zone de la pointe de l'aiguille de lancette, **caractérisée en ce que** la pointe de l'aiguille de lancette est enrobée d'une matière élastique avant son utilisation sans laisser subsister un espace creux.

2. Lancette selon la revendication 1, **caractérisée en ce que** la matière élastique est un élastomère thermoplastique.

3. Lancette selon l'une quelconque des revendications 1 à 2, **caractérisée en ce que** la matière élastique est stabilisée avec une matière rigide.

4. Jeu de lancettes contenant au moins deux lancettes selon l'une quelconque des revendications 1 à 3, qui sont reliées l'une à l'autre.

5. Jeu de lancettes selon la revendication 4, **caractérisé en ce que** les lancettes sont reliées l'une à l'autre via la matière élastique.

6. Magasin de lancettes contenant au moins deux lancettes, qui contiennent respectivement une aiguille de lancette comprenant une pointe et qui viennent se loger respectivement dans des chambres individuelles, indépendantes les unes des autres, du magasin de lancettes, chaque chambre présentant au moins une ouverture pour la sortie de la pointe de l'aiguille de lancette, l'ouverture en question de la chambre étant obturée par une matière élastique, de telle sorte que la matière élastique peut être percée par la pointe de l'aiguille de lancette lors de la piqûre, **caractérisé en ce que** la matière élastique, après le retrait de la pointe de l'aiguille de lancette dans la chambre, se referme à nouveau de manière étanche.

7. Magasin de lancettes selon la revendication 6, **caractérisé en ce que** la matière élastique est un élastomère thermoplastique.

8. Utilisation d'une matière élastique à titre de constituant d'une lancette selon l'une quelconque des revendications 1 à 5 pour le maintien de la stérilité au moins de la pointe d'une aiguille de lancette à l'état non utilisé.

9. Utilisation d'une matière élastique à titre de constituant d'une lancette selon l'une quelconque des revendications 6-7 pour le maintien de la stérilité au moins de la pointe d'une aiguille de lancette à l'état non utilisé.

10. Utilisation selon la revendication 9 ou 10, dans laquelle la matière élastique est utilisée en outre pour la protection hygiénique au moins de la pointe d'une aiguille de lancette à l'état utilisé.

11. Utilisation selon la revendication 8 ou 9, **caractérisée en ce que** la matière élastique est un élastomère thermoplastique.
